Europäisches Patentamt

European Patent Office

Office européen des brevets

(11) Numéro de publication: **0 345 242**
A2

## DEMANDE DE BREVET EUROPEEN

(21) Numéro de dépôt: 89870082.8

(22) Date de dépôt: 02.06.89

(51) Int. Cl.⁴: **C 12 N 15/00**
C 12 N 7/00, C 12 N 5/00,
C 12 N 1/18, A 61 K 39/21,
G 01 N 33/569

(30) Priorité: 03.06.88 US 202271

(43) Date de publication de la demande:
06.12.89 Bulletin 89/49

(84) Etats contractants désignés:
AT BE CH DE ES FR GB GR IT LI LU NL SE

(71) Demandeur: SMITHKLINE BIOLOGICALS S.A.
Rue du Tilleul, 13
B-1320 Genval Rixensart (BE)

(72) Inventeur: Gheysen, Dirk
Daloensdelle, 48
B-1900 Overijse (BE)

Jacobs, Eric
Avenue des Erables, 3
B-1320 Genval (Rixensart) (BE)

(74) Mandataire: Tasset, Gérard
Smithkline Biologicals s.a. Rue de l'Institut 89
B-1330 Rixensart (BE)

Revendications pour les Etats contractants suivants: ES + GR.

(54) Expression de protéines gag de rétrovirus dans les cellules eucaryotes.

(57) La protéine précurseur gag de rétrovirus est exprimée dans des cellules eucaryotes par techniques d'ADN recombinant et employée pour induire une immunoprotection chez les humains présentant un risque d'exposition à HIV et pour diagnostiquer une exposition.

## Description

### Domaine de l'Invention

Cette invention est relative à l'expression des protéines dans les cellules eucaryotes. Elle concerne plus particulièrement l'expression de la protéine précurseur gag du virus de l'immunodéficience.

### Arrière-plan de l'Invention

Les rétrovirus, c'est-à-dire les virus de la famille des Retroviridae, sont une grande famille de virus icosohédraux enveloppés d'environ 150 nm présentant une nucléocapside et dont le matériel génétique est de l'ARN. La famille comprend les oncovirus comme les virus du sarcome et de la leucémie, les virus de l'immunodéficience et les lentivirus.

Le virus de la déficience immunitaire humaine (HIV), l'agent étiologique du syndrome de la déficience immunitaire acquise (SIDA) et des désordres qui y sont apparentés, est un membre de la famille des Rétroviridae. Il existe différents isolats de HIV parmi lesquels le virus T-lymphotropique humain de type III (HTLV-III), le virus de la lymphadénopathie (LAV) et les rétrovirus associés au SIDA (ARN) qui ont été groupés dans le type 1. Les virus associés à la déficience immunitaire comprennent HIV de type 2 dont l'association au SIDA en Afrique occidentale a été récemment mise en évidence. D'autres virus de la déficience immunitaire comprennent les virus SIV (virus du singe) tels que SIV$_{mac}$-BK28.

La caractérisation moléculaire du génome de HIV a démontré que le virus possède la même organisation gagpol-env globale que d'autres rétrovirus. De plus, il contient au moins 5 gènes qui ne se retrouvent pas dans d'autres rétrovirus : sor, tat3, art/trs, 3'orf et R. La région gag encode au moins 3 protéines structurelles ("core"), p17, p24 et p16 qui sont généraes par clivage d'une protéine précurseur gag de 55 kilodaltons par la protéase de HIV. La protéase est encodée par le gène pol.

Des rapports récents ont montré que des anticorps aux protéines gag de HIV, p17, p24 et p16 sont présents dans les séra humains d'individus infectés aux Etats-Unis d'Amérique et en Europe et que les anticorps apparaissent rapidement après infection. La présence de ces anticorps décline au fur et à mesure que l'individu progresse vers le SIDA.

La protéine gag p17 avec sa localisation submembranaire (Gelderblom et al, Virology 156:171 (1987) est bien positionné pour être en contact étroit avec la protéine transmembranaire gp41 et la membrane virale. Elle pourrait également jouer un rôle dans les changement de conformation impliqués dans le processus d'entrée et de désenrobage du virus. De plus, on a démontré que gag p17 a un N-terminus myristylé. La myristylation jouerait un rôle important dans l'assemblage du virion et dans la localisation de composants du virus à la membrane du plasma.

Malgré de gros efforts de recherche dans le domaine du SIDA, on continue à avoir besoin de réactifs de diagnostic qui peuvent être utilisés pour contrôler la progression de la maladie et d'agents qui peuvent éviter une infection primaire, tels que via une immunisation et d'agents qui peuvent prévenir ou empêcher une infection secondaire tels que par une transmission de cellule à cellule ou par infection du virus libre.

### Résumé de l'Invention

Un aspect de cette invention est une molécule d'ADN recombinant pour l'expression de la protéine précurseur gag dans des cellules eucaryotes qui comprend une séquence codant liée de manière opératoire à une région de régulation qui fonctionne dans la cellule hôte.

Des aspects voisins de cette invention sont des cellules hôtes comprenant la molécule d'ADN recombinant et leurs cultures.

D'autres aspects de cette invention sont la protéine précurseur gag produite par les cellules hôtes de l'invention, y compris une particule comprenant la protéine précurseur gag semblable au "core" de HIV.

D'autres aspects encore de cette invention sont un procédé pour produire la molécule d'ADN recombinant et la cellule hôte de l'invention, un procédé pour produire la protéine précurseur gag et les particules de l'invention ainsi que les compositions et méthodes qui s'y rattachent.

Ces aspects de l'invention et d'autres encore sont amplement décrits dans la description et les Exemples qui suivent.

### Description Détaillée de l'Invention

Il a maintenant été trouvé que la protéine précurseur rétrovirale gag peut être exprimée dans des cellules eucaryotes recombinantes et qu'une telle expression peut résulter en une production de l'entièreté de la protéine précurseur gag sans l'usage de séquences d'ADN pol et sans l'usage de séquences 5' non traduites.

Comme exemples de telles cellules il y a les cellules d'eucaryotes inférieurs telles que les cellules de levure, de moisissures et d'animaux, y compris les cellules d'insectes telles que les cellules de Drosophiles ou de Lépidomtères; les lignées cellulaires de mammifères; les cellules primaires de mammifères et les insectes et animaux transgéniques.

Il a également été trouvé, de manière inattendue, que la protéine précurseur gag peut former des particules qui ressemblent, en grandeur et autres caractéristiques physiques et en antigénicité, aux particules gag authentiques formées à la surface des cellules humaines infectées. Durant un cycle d'infection rétrovirale naturelle, il apparaît que la protéine précurseur gag, connue comme p55 dans le cas de HIV, est tout d'abord assemblée en particules immatures comprenant de manière prépondérante la protéine gag dans son entièreté. Ces particules gag peuvent être désignées comme pré-particules de "core" ou comme particules de noyau non matures. Pendant la maturation virale, le précurseur est clivé en protéines sousunitaires connues, dans le cas de HIV, comme p17, p24 et p16. Ces particules gag, maintenant constituées en majeure partie de p17, p24 et p16, peuvent être mentionnées comme particules de "core" ou particules matures de "core". Pendant la maturation virale également, apparemment pendant le processus de bourgeonnement, la membrane virale se forme autour des particules du "pré-core" ou du "core". Comme le montrent les Exemples ci-après, le précurseur gag de HIV exprimé dans des cellules recombinantes de Lépidoptères en utilisant un système d'expression Baculovirus forme en majeure partie des agrégats ou des paquets sous forme de particules qui ont des propriétés physiques et biologiques et des dimensions similaires à celles du "core" des particules de HIV formées naturellement à la surface des cellules humaines infectées. Les particules de l'invention comprennent de manière prédominante la protéine précurseur gag (plus de 90 % des protéines présentes dans les par ticules. Elles sont reconnues après un bref traitement avec du Triton X100 par des anticorps monoclonaux (MABs) anti-p17, MABs anti-p24 et MABs anti-p16 en plus de leur reconnaissance par des anticorps polyclonaux anti-gag provenant de sera de patients infectés. Les particules, parce qu'elles sont préparées par techniques d'ADN recombinant comme décrit ici, sont dépourvues des fonctions virales requises pour la maturation et la réplication du virus, spécialement de l'ARN viral et, de préférence, également des fonctions de transcriptase inverse.

Les cellules eucaryotes recombinantes de l'invention sont construites pour exprimer la protéine précurseur gag par introduction dans les cellules de la molécule d'ADN recombinant de l'invention. La molécule d'ADN recombinant de l'invention comprend une région codant pour la protéine précurseur gag liée de manière opératoire à un élément de régulation qui fonctionne dans les cellules hôtes choisies. Sous un aspect de cette invention, il a été trouvé que d'autres fonctions de HIV ne sont pas requises pour l'expression de la protéine précurseur gag et pour la formation de la particule ressemblant au 'pré-core'. Les séquences d'ADN codant pour d'autres fonctions, p.ex. pour des fonctions d'amplification, fonctions de marqueurs de sélection ou de maintenance peuvent également être associées à la molécule d'ADN recombinant de l'invention.

Une région d'ADN codant pour la protéine précurseur gag peut être préparée de n'importe lequel des différents clones génomiques d'isolat infectieux du virus de l'immunité déficitaire rapportés dans la littérature. Voir, par exemple Shaw et al., Science 226:1165 (1984); Kramer et al., Science 231:1580 (1986). Alternativement, un clone génomique du virus de l'immunité déficitaire peut être préparé de virus isolés de spécimens cliniques par technique de clonage d'ADN standard. Voir, par exemple, Gallo et al., Brevet 4 520 113 des Etats-Unis d'Amérique; Montagnier et al., Brevet 4 708 818 des Etats-Unis d'Amérique. Ayant cloné un fragment du génome qui comprend la région codante gag, une région qui code seulement pour le précurseur gag peut être préparée par restriction de l'ADN de manière à isoler une portion de la région codante de l'ADN et reconstruction des portions restantes par l'utilisation d'oligonucléotides synthétiques, comme décrit dans les Exemples ci-après. Alternativement, un fragment plus important comprenant la région codante gag ou des séquences additionnelles peut être coupé par l'utilisation d'exonucléases. Dans un autre procédé alternatif encore, l'entièreté de la région codante peut être synthétisée en utilisant des synthétiseurs d'ADN automatisés standard par la synthèse de fragments de la région codante et en les liant ensemble pour former une région codante complète. Bien que l'usage d'une séquence codante dépourvue des séquences flanquantes naturelles présentes en 5' et en 3' soit préféré, la fusion de la séquence codante à d'autres séquences du virus de l'immunité déficitaire, p.ex. séquences de la protéine du manteau, n'est pas écartée des aspects préférés.

A titre d'exemple, une région codant pour la protéine précurseur gag de HIV a la séquence suivante :

```
  1 ATG GGT GCG AGA GCG TCA GTA TTA AGC GGG GGA GAA   36
    Met Gly Ala Arg Ala Ser Val Leu Ser Gly Gly Glu

 37 TTA GAT CGA TGG GAA AAA ATT CGG TTA AGC CCA GGG   72
    Leu Asp Arg Trp Glu Lys Ile Arg Leu Arg Pro Gly

 73 GGA AAG AAA AAA TAT AAA TTA AAA CAT ATA GTA TGG  108
    Gly Lys Lys Lys Tyr Lys Leu Lys His Ile Val Trp

109 GCA AGC AGG GAG CTA GAA CGA TTC GCA GTT AAT CCT  144
    Ala Ser Arg Glu Leu Glu Arg Phe Ala Val Asn Pro

145 GGC CTG TTA GAA ACA TCA GAA GGC TGT AGA CAA ATA  180
    Gly Leu Leu Glu Thr Ser Glu Gly Cys Arg Gln Ile

181 CTG GGA CAG CTA CAA CCA TCC CTT CAG ACA GGA TCA  216
    Leu Gly Gln Leu Gln Pro Ser Leu Gln Thr Gly Ser

217 GAA GAA CTT AGA TCA TTA TAT AAT ACA GTA GCA ACC  252
    Glu Glu Leu Arg Ser Leu Tyr Asn Thr Val Ala Thr

253 CTC TAT TGT GTG CAT CAA AGG ATA GAG ATA AAA GAC  288
    Leu Tyr Cys Val His Gln Arg Ile Glu Ile Lys Asp

289 ACC AAG GAA GCT TTA GAC AAG ATA GAG GAA GAG CAA  324
    Thr Lys Glu Ala Leu Asp Lys Ile Glu Glu Glu Gln

325 AAC AAA AGT AAC AAA AAA GCA CAG CAA GCA GCA GCT  360
    Asn Lys Ser Asn Lys Lys Ala Gln Gln Ala Ala Ala

361 GAC ACA GGA CAC AGC AGT CAG GTC AGC CAA AAT TAC  396
    Asp Thr Gly His Ser Ser Gln Val Ser Gln Asn Tyr

397 CCT ATA GTG CAG AAC ATC CAG GGG CAA ATG GTA CAT  432
    Pro Ile Val Gln Asn Ile Gln Gly Gln Met Val His

433 CAG GCC ATA TCA CCT AGA ACT TTA AAT GCA TGG GTA  468
    Gln Ala Ile Ser Pro Arg Thr Leu Asn Ala Trp Val

467 AAA GTA GTA GAA GAG AAG GCT TTC AGC CCA GAA GTA  504
    Lys Val Val Glu Glu Lys Ala Phe Ser Pro Glu Val

505 ATA CCC ATG TTT TCA GCA TTA TCA GAA GGA GCC ACC  540
    Ile Pro Met Phe Ser Ala Leu Ser Glu Gly Ala Thr
```

4

541 CCA CAA GAT TTA AAC ACC ATG CTA AAC ACA GTG GGG 576
    Pro Gln Asp Leu Ash Thr Met Leu Asn Thr Val Gly

577 GGA CAT CAA GCA GCC ATG CAA ATG TTA AAA GAG ACC 612
    Glv His Gln Ala Ala Met Gln Met Leu Lys Glu Thr

613 ATC AAT GAG GAA GCT GCA GAA TGG GAT AGA GTA CAT 648
    Ile Asn Glu Glu Ala Ala Glu Trp Asp Arg Val His

649 CCA GTG CAT GCA GGC CCT ATT GCA CCA GGC CAG ATG 684
    Pro Val His Ala Gly Pro Ile Ala Pro Gly Gln Met

685 AGA GAA CCA AGG GGA AGT GAC ATA GCA GGA ACT ACT 720
    Arg Glu Pro Arg Gly Ser Asp Ile Ala Gly Thr Thr

721 AGT ACC CTT CAG GAA CAA ATA GGA TGG ATG ACA AAT 756
    Ser Thr Leu Gln Glu Gln Ile Gly Trp Met Thr Asn

757 AAT CCA CCT ATC CCA GTA GGA GAA ATT TAT AAA AGA 792
    Asn Pro Pro Ile Pro Val Gly Glu Ile Tyr Lys Arg

793 TGG ATA ATC CTG GGA TTA AAT AAA ATA GTA AGA ATG 828
    Trp Ile Ile Leu Gly Leu Asn Lys Ile Val Arg Met

829 TAT AGC CCT ACC AGC ATT CTG GAC ATA AGA CAA GGA 864
    Tyr Ser Pro Thr Ser Ile Leu Asp Ile Arg Gln Gly

865 CCA AAA GAA CCT TTT AGA GAC TAT GTA GAC CGG TTC 900
    Pro Lys Glu Pro Phe Arg Asp Tyr Val Asp Arg Phe

901 TAT AAA ACT CTA AGA GCC GAG CAA GCT TCA CAG GAG 936
    Tyr Lys Thr Leu Arg Ala Glu Gln Ala Ser Gln Glu

937 GTA AAA AAT TGG ATG ACA GAA ACC TTG TTG GTC CAA 972
    Val Lys Asn Trp Met Thr Glu Thr Leu Leu Val Gln

```
1405 GCC CCA CCA GAA GAG AGC TTC AGG TCT GGG GTA GAG 1440
     Ala Pro Pro Glu Glu Ser Phe Arg Ser Gly Val Glu

1441 ACA ACA ACT CCC CCT CAG AAG CAG GAG CCG ATA GAC 1476
     Thr Thr Thr Pro Pro Gln Lys Gln Glu Pro Ile Asp

1477 AAG GAA CTG TAT CCT TTA ACT TCC CTC AGA TCA CTC 1512
     Lys Glu Leu Tyr Pro Leu Thr Ser Leu Arg Ser Leu

 973 AAT GCG AAC CCA GAT TGT AAG ACT ATT TTA AAA GCA 1008
     Asn Ala Asn Pro Asp Cys Lys Thr Ile Leu Lys Ala

1009 TTG GGA CCA GCG GCT ACA CTA GAA GAA ATG ATG ACA 1044
     Leu Gly Pro Ala Ala Thr Leu Glu Glu Met Met Thr

1045 GCA TGT CAG GGA GTA GGA GGA CCC GGC CAT AAG GCA 1080
     Ala Cys Gln Gly Val Gly Pro Gly Gly His Lya Ala

1081 AGA GTT TTG GCT GAA GCA ATG AGC CAA GTA ACA AAT 1116
     Arg Val Leu Ala Glu Ala Met Ser Gln Val Thr Asn

1117 ACA GCT ACC ATA ATG ATG CAG AGA GGC AAT TTT AGG 1152
     Thr Ala Thr Ile Met Met Gln Arg Gly Asn Phe Arg

1153 AAC CAA AGA AAG ATG GTT AAG TGT TTC AAT TGT GGC 1188
     Asn Gln Arg Lys Met Val Lys Cys Phe Asn Cys Gly

1189 AAA GAA GGG CAC ACA GCC AGA AAT TGC AGG GCC CCT 1224
     Lys Glu Gly His Thr Ala Arg Asn Cys Arg Ala Pro

1225 AGG AAA AAG GGC TGT TGG AAA TGT GGA AAG GAA GGA 1260
     Arg Lys Lys Gly Cys Trp Lys Cys Gly Lys Glu Gly

1261 CAC CAA ATG AAA GAT TGT ACT GAG AGA CAG GCT AAT 1296
     His Gln Met Lys Asp Cys Thr Glu Arg Gln Ala Asn

1297 TTT TTA GGG AAG ATC TGG CCT TCC TAC AAG GGA AGG 1332
     Phe Leu Gly Lys Ile Trp Pro Ser Tyr Lys Gly Arg

1333 CCA GGG AAT TTT CTT CAG AGC AGA CCA GAG CCA ACA 1368
     Pro Gly Asb Phe Leu Gln Ser Arg Pro Glu Pro Thr

1369 GCC CCA CCA TTT CTT CAG AGC AGA CCA GAG CCA ACA 1404
     Ala Pro Pro Phe Leu Gln Ser Arg Pro Glu Pro Thr
```

Un grand nombre de cellules eucaryotes et de systèmes d'expression sont disponibles pour l'expression de protéines hétérologues. Les plus largement utilisées parmi eux sont les systèmes de mammifères, insectes et levures, bien que l'invention ne leur soit pas limitée. De manière typique, ces systèmes emploient une molécule d'ADN recombinant comprenant une séquence codant pour le gène intéressant lié de manière opératoire à un élément de régulation, un marqueur de sélection et, dans certains cas, des fonctions de maintenance comme une origine de réplication. Un élément de régulation est une région ou un ensemble de régions d'ADN qui comprend les fonctions nécessaires ou désirables pour la transcription et la traduction. De manière typique, la région de régulation comprend un promoteur pour fixation de polymérase d'ARN et initiation de transcription.

Parmi les cellules d'insectes qui peuvent être utilisées dans l'invention il y a les cellules de Drosophiles et de Lépidoptères. Parmi les cellules de Drosophiles utiles il y a les cellules S1, S2, S3 et KC-O et D. hydei. Voir, par exemple, Schneider et al., J. Embryol. Exp. Morph. 27:353 (1972); Schulz et al., Proc. Natl. Acad. Sci. USA 83:9428 (1986); Sinclair et al., Mol. Cell. Biol. 5:3208 (1985). Les cellules de Drosophiles sont transfectées par techniques standard, y compris la précipitation au phosphate de calcium, la fusion cellulaire, l'électroporation et la transfection virale. Les cellules sont cultivées suivant les procédures de culture cellulaire standard dans une variété de milieux nitritifs, y compris, p.ex., le milieu M3 qui consiste en un ensemble d'acides aminés essentiels et de sels équilibrés. Voir Lindquist, DIS 58:163 (1982).

Parmi les promoteurs connus pour être utiles chez les Drosophiles, il y a les promoteurs de cellules de mammifères aussi bien que les promoteurs de Drosophiles, ces derniers étant préférés. Comme exemples de promoteurs de Drosophiles utiles il y a le promoteur de métallothionéine de Drosophile, le promoteur de la protéine de choc thermique 'heatshock' de 70 kilodaltons (HSP70) et le LTR COPIA. Voir, par exemple, Di Nocera et al., Proc. Natl. Acad. Sci. USA 80:7095 '1983); McGarry et al., Cell 42:903 (1985). En pratique, une cassette d'expression comprenant la séquence codant pour gag et un élément de régulation peut être cloné dans un vecteur de clonage bactérien dans le but de propager l'ADN avant la transfection des cellules animales.

Dans les agents préférés de cette invention, le précurseur gag de HIV est exprimé dans des cellules de Lépidoptères pour produire des particules gag immunogéniques. Pour l'expression de la protéine précurseur gag dans des cellules de Lépidoptères, on préfère utiliser un système d'expression Baculovirus. Dans un tel système, une cassette d'expression comprenant la séquence codant pour gag et l'élément de régulation est placée dans un vecteur de clonage standard dans un but de propagation. Le vecteur recombinant est alors cotransfecté dans des cellules de Lépidoptères avec de l'ADN d'un type de Baculovirus sauvage. Les virus recombinants résultant de la recombinaison homologue sont alors sélectionnés et purifiés sur plaque en substance comme décrit par Summers et al., TAES Bull. NR 1555, Mai, 1987.

Parmi les cellules de Lépidoptères utiles, il y a les cellules de Trichloplusia ni, Spodoptera Frugiperda, Heliothis zea, Autographica californica, Rachiplusia ou, Galleria melonella, Mandica Sexta ou autres cellules qui peuvent être infectées par des Baculovirus, y compris le virus de polyhédrose nucléaire (VPN), les virus à nucléocapside unique (VNCU). Les Baculovirus préférés sont VPN ou VNCM parce qu'ils contiennent le promoteur du gène de polyhédrine qui est fortement exprimé dans les cellules infectées. Particulièrement exemplifié ci-après est le virus VNCM de Autographica californica (AcVNCM). Cependant, d'autres virus VNCM et VNCU peuvent également être employés comme le virus du ver à soie, Bombyx mori. Les cellules de Lépidoptères sont co-transfectés avec de l'ADN comprenant la cassette d'expression de l'invention et avec l'ADN d'un Baculovirus infectieux par techniques de transfection standard, comme exposé plus haut. Les cellules sont cultivées suivant les techniques de culture cellulaire standard dans un bon nombre de milieux nitritifs, y compris, par exemple, TC100 (Gibco Europe; Gardiner et al., J. Inverteb. Pathol. 25:363 (1975) additionné de 10 % de sérum foetal de veau (SFV). Voir Miller et al., dans Setlow et al. ed., Genetic Engineering : Principles and Methods, Volume 8, New York, Plenum, 1986, pages 277-298.

La production dans les cellules d'insectes peut également être effectuée par infection de larves d'insectes. Par exemple, le précurseur peut être produit dans des chenilles de Trichoplusia ni en continuant à donner comme nourriture le Baculovirus recombinant avec des traces de Baculovirus de type sauvage et en extrayant ensuite le précurseur gag de l'hémolymphe après environ 2 jours.

Parmi les promoteurs utilisables dans les cellules de Lépidoptères il y a les promoteurs d'un génome de Baculovirus. Le promoteur du gène de la polyhédrine est préféré parce que la protéine polyhédrine est naturellement sur-exprimée par rapport aux autres protéines de Baculovirus. On préfère le promoteur du gène de polyhédrine du virus AcVNCM. Voir Summers et al., TAES Bull. Nr 1555, Mai 1987; Smith et al., EP-A-127839, Smith et al., Proc. Natl. Acad. Sci. USA 82:8404 (1985) et Cochran EP-A-228036.

Pour l'expression dans des cellules de mammifères, la cassette d'expression est, de la même manière, clonée dans un vecteur de clonage et alors utilisée pour transfecter les cellules de mammifères. Le vecteur comprend, de préférence, des fonctions ADN additionnelles pour la sélection et/ou l'amplification, p.ex. une cassette de résistance à la néomycine pour sélection G 418. D'autres fonctions, telles que pour l'augmentation de la transcription peuvent également être employées. D'autres fonctions encore peuvent être comprises dans le vecteur pour une stabilité de la maintenance épisomale, si on le désire, telles que les fonctions de maintenance du virus du papillome bovin. Le vecteur cellulaire de mammifère peut également être un virus recombinant, comme un recombinant de la vaccine ou d'autre pox virus. Voir, p.ex., Paoletti et al., brevet 4·603 112 des Etats-Unis d'Amérique; Paoletti et al., Proc. Natl. Acad. Sci. U.S. 81:193 (1984).

Parmi les cellules de mammifères qui sont utiles il y a les cellules de l'ovaire de hamster chinois (CHO), NIH3T3, COS-7, CVI, du myélome de la souris ou du rat, HAK, Vero, HeLa, les cellules diploïdes humaines, comme MRC-5 et WI38 ou les lignées cellulaires du lymphone du poulet. La transfection et la culture cellulaire sont effectués par techniques standard. La production dans les cellules de mammifères peut également être effectuée par expression dans des animaux transgéniques. Par exemple, le précurseur gag peut être exprimé en utilisant un promoteur de caséine et purifié à partir de lait.

Parmi les promoteurs utiles dans les lignées cellulaires de mammifères ou les cellules primaires de mammifères il y a les promoteurs de gènes initiaux et tardifs, le promoteur de la métallothionéine, les LTR viraux comme le LTR du sarcome de Rous, le LTR du virus du sarcome de Moloney ou le LTR du virus de la tumeur mammaire de la souris (VTMS) ou le promoteur tardif principal de l'adénovirus et les promoteurs hybrides comme un virus hybride BK et le promoteur tardif principal d'adénovirus. La région de régulation peut également comprendre des fonctions en aval, comme des fonctions pour l'adénylation ou d'autres fonctions, comme des séquences pour amplifier la transcription.

Parmi les levures qui peuvent être employées dans la mise en pratique de l'invention il y a celles des genres Hansenula, Pichia, Kluveromyces, Schizosaccharomyces, Candida et Saccharomyces. La levure hôte préférée est le Saccharomyces cerevisiae. Parmi les promoteurs utiles, il y a le promoteur inductible de cuivre (CUPI), les promoteurs de gènes glycolytiques, p.ex. TDH3, PGK et ADH, et les promoteurs PHO5 et ARG3. Voir, par exemple, Miyanohara et al., Proc. Natl. Acad. Sci. USA 80:1 (1983), Mellor et al, Gene 24:1 (1983); Hitzemann et al., Science 219:620 (1983); Cabezon et al, Proc. Natl. Acad. Sci. USA 81:6594 (1984).

Dans le cas de particules de protéine précurseur gag produites suivant cette invention, il est entendu que, bien qu'on préfère des particules comprenant le précurseur gag, on peut également préparer des particules contenant des dérivés du précurseur gag natif. Par exemple, un ou plusieurs nucléotides ou acides aminés indiqués dans la séquence ci-dessus peuvent être supprimés, substitués ou ajoutés sans affecter défavorablement de manière sensible la réactivité immunogénique croisée avec mes épitopes gag authentiques. En d'autres termes, de tels dérivés peuvent être immunologiquement similaires à des particules gag authentiques. De tels dérivés, bien qu'ils puissent comprendre des acides aminés provenant d'autres régions, y compris des régions immunogéniques du génome de HIV, n'encodent pas d'autres fonctions de HIV, comme la fonction protéase de la région pol ou la fonction transcriptase inverse. De plus, de tels dérivés retiennent la faculté de former des particules en culture de cellules d'insectes comme il est décrit ici. Dans ce cas, il est à la portée de l'homme du métier de préparer des particules gag qui comprennent des protéines hybrides ayant un ou plusieurs épitopes supplémentaires aux épitopes gag. De tels épitopes supplémentaires peuvent être d'origine HIV ou dérivés d'autres organismes pathogènes, p.ex. virus de l'hépatite B ou virus de l'herpes.

La protéine précurseur gag est exprimée dans la forme liée à la membrane (particules et amas de particules enrobées de membranes). Elle est isolée du milieu conditionné par techniques standard d'isolement et purification des protéines. On peut ajouter des détergents afin de libérer la protéine du matériel membranaire de la cellule. Après traitement avec un détergent, p.ex. du Triton X100, un Tween ou du sulfate de dodécyl sodium (SDS), la protéine ou les particules peuvent être purifiées par une série d'étapes d'ultrafiltration, étapes d'ultracentrifugation, précipitation sélective avec, p.ex. du sulfate d'ammonium ou PEG, gradient de densité, centrifugation dans CsCl ou gradient de saccharose et/ou stades chromatographiques comme chromatographie d'affinité, chromatographie d'immunoaffinité, HPLC, HPLC en phase inversée, échange cationique ou anionique, chromatographie d'exclusion de dimension et focalisation isoélectrique préparative. Pendant ou après purification, la protéine ou les particules peuvent être traitées p.ex. avec du formaldéhyde, du glutaraldéhyde ou du NAE pour augmenter la stabilité ou l'immunogénicité. A la vue de la découverte décrite ici que le précurseur gag peut former des particules immunogènes en absence d'autres fonctions virales, on croit que, lorsque le précurseur gag est exprimé et purifié sous forme non-particulaire, il peut causer la formation de particules in vitro, comme il a été montré que c'est le cas pour l'antigène de surface de l'hépatite B après expression dans la levure. Voir, p.ex., EP-A-135435. De telles particules de protéine précurseur gag sont comprises dans la portée de cette invention.

La protéine précurseur gag de HIV et les particules produites suivant cette invention sont utiles comme agents de diagnostic pour la détection d'une exposition à HIV. La protéine et les particules sont également utiles dans des vaccins pour la prévention de l'infection ou pour l'inhibition ou la prévention d'une progression de la maladie.

Les exemples qui suivent sont illustratifs mais non limitatifs de l'invention. Les enzymes de restriction et autres agents ont été utilisés en substance en suivant les instructions des vendeurs.

Exemples

Exemple 1. Construction du Vecteur

pRIT12982 (DT 12-16) est un vecteur qui comprend une séquence de 1305 paires de bases (pb) codant pour la région N-terminale de la protéine précurseur gag. Il a été préparé par ligation d'un fragment ClaI-BglII de la région codant pour la protéine précurseur gag dérivée d'un clone génomique de HIV (Shaw et al. Science 226:1165 (1984) avec un oligonucléotide synthétique ayant la séquence codante N-terminale de la protéine précurseur gag. L'oligonucléotide a la séquence :

```
5' C ATG GGT GCT AGA GCT TCC GTG TTG TCC GGT GGT GAA TTG GAT 3'
       CCA CGA TCT CGA AGG CAC AAC AGG CCA CCA CTT AAC CTA GC
   NcoI                                                    ClaI
```

pRIT12983 est un vecteur qui comprend une région de 250 pb qui code pour la portion C-terminale de la protéine précurseur gag. Il a été préparé par ligation d'un fragment BglII-MaeIII de la région codant pour le précurseur gag dérivée d'un clone génomique de HIV avec un oligonucléotide synthétique ayant la séquence codante C-terminale de la protéine précurseur gag. L'oligonucléotide a la séquence :

```
                  STOP
5' G RCA CAA TAA AGA TAG GAT CC  3'
       TT ATT TCT ATC CTA GGA GCT
   MaeIII                  XhoI
```

Le fragment BamHI(NcoI)-Bg1II de 1305 paires de bases (pb) a été lié au fragment BglII-TAA-BamHI-XhoI de 250 pb provenant de pRIT 12983 dans pUC12 qui avait été préalablement coupé avec BamHI et SalI. Le plasmide résultant, identifié comme pRIT13001 contient par conséquent l'entièreté de la région codant pour la protéine précurseur gag sur une cassette BamHI(NcoI)-BamHI.

Un vecteur d'expression de baculovirus a été préparé en insérant le fragment BamHI provenant de pRIT13001 au site BamHI de pAC373. Voir Smith et al., Proc. Natl. Acad. Sci. USA 82:8404 (1985). pAC373 est un vecteur de transfert de Baculovirus contenant un gène de polyhédrine modifié dans lequel un gène étranger peut être cloné à un site BamHI et exprimé sous le contrôle du fort promoteur de la polyhédrine. Voir Summers et al., Texas Agricultural Exp. Station Bulletin NR 1555 (Mai 1987). Un dérivé du plasmide pAC373 ayant une petite délétion loin en amont du promoteur de la polyhédrine a également été utilisé comme vecteur d'expression. La légère modification ne s'est pas avérée affecter in vitro l'expression ou le développement du virus recombinant. L'insertion de la séquence codant pour gag dans le vecteur de Baculovirus a eu pour résultat le plasmide pRIT13003.

Un vecteur d'expression de cellule de mammifère a été préparé par ligation du fragment BamHI de pRIT13001 en aval du promoteur tardif de SV40 dans pSV529 (Gheysen et al., J. Mol. Appl. Genet. 1:384 (1982). Ce vecteur est identifié comme pRIT13002.

Un vecteur d'expression de levure a été préparé comme suit. Un fragment NcoI-BglII a été isolé de pRIT12982 et inséré dans un plasmide de levure en aval du promoteur ARG3 (voir Cabezon et al., Proc. Natl. Acad. Sci. USA 81:6594 (1984) pour donner naissance au vecteur pRIT12984 (DT14-20). La portion C-terminale de la protéine précurseur gag a été isolée de pRIT12983 sous forme d'un fragment BglII-BamHI et a été inséré au site BglII de pRIT12984 pour donner le vecteur de levure pRIT12985 (DT16-26). pRIT12985 comprend donc une séquence codant pour l'entièreté du précurseur gag, dépourvue d'autres séquences de HIV, liée de manière opératoire au promoteur ARG3. De plus, il comprend les fonctions de réplication provenant du vecteur de 2 microns de la levure et le gène URA3 comme marqueur de sélection.

Exemple 2. Expression dans les cellules d'insectes.

Des Baculovirus recombinants transfectés avec pRIT13003 ont été préparés en substance comme décrit par Summers et al., TAES Bull. NR 1555, Mai 1987, cité plus haut.

Des cellules de Spodoptera frugiperda (s.f.) ont été co-transfectées avec respectivement 1 µg de Baculovirus AC VNCM de type sauvage (ts) et 50 µg de plasmide pRIT13003. Les particules de virus résultantes ont été obtenues en recueillant les surnageants. Les milieux contenant les virus ont été utilisés pour infecter des cellules de S.f. dans un test sur plaque. Une infection subséquente de cellules de S.f. en utilisant les particules virales qui comprennent à la fois de l'ADN de AcNPV et de l'ADN recombiné avec l'ADN encodant la protéine précurseur gag p55 a donné des cellules exprimant la protéine gag p55 au lieu de la protéine polyhédrine.

Les "plaques claires" (fréquence 0,1 - 0,01 %) obtenues dans l'essai sur plaque ont ensuite été criblées par hybridation sur filtre avec une sonde spécifique pour gag. Les plaques qui s'hybridaient à la sonde gag ont été enregistrées et ensuite purifiées (sur plaque) (2-3 fois) avant de générer un stock de virus; le stock de virus a alors été testé par ELISA. Des cellules de S.f. ont alors été infectées avec ces stocks de virus contenant du gag recombinant à un facteur de multiplicité d'infection (FMI) de 1-10 et, après 24 hres, 48hres, 3 jours et 5 jours, des aliquo tes de milieu conditionné (surnageant) et/ou de cellules ont été traitées avec du Triton X100 jusqu'à concentration finale de 1 % et testées.

La protéine précurseur gag synthétisée dans les cellules d'insecte infectées a été observée dans des "Western blots" en utilisant des anticorps polyclonaux p55 ou de l'antisérum provenant d'un pool de patients SIDA (Zaire). Une bande prédominante à un poids moléculaire (PM) de 54 kilodaltons (kd) a été observée avec tous les séra testés et avec les antiséra polyclonaux de p55. Une bande à un PM de 54 kd a également été détectée en testant du milieu conditionné après 48 heures, 3 jours et 5 jours. Des bandes à un PM de 49 kd et à un PM de 47 kd (mineure) et une bande à un PM de 30 kd ont pu également être détectée lors de l'analyse des extraits cellulaires. Cette dernière bande avec un PM de 30 kd n'est détectée qu'avec les anticorps polyclonaux de p55 et pas avec le sérum de personnes infectées par le SIDA. Il a été observé que au moins 10 fois plus d'"épitopes p55" étaient exprimés dans les cellules de S.f. que dans des cellules Molt infectées avec HIV (Molt/HTLV-III) et environ 80 fois plus d'"épitopes" p55 étaient présents dans le milieu conditionné de cellules de S.f. infectées avec un virus à gag recombinant que dans le milieu conditionné de cellules Molt/HTLV-III.

Dans l'expérimentation d'un second essai, l'ultracentrifugation (100.000 x g, 1 hre) des milieux conditionnés (2 ml à 200 ml) de 48 heures, 3 jours et 5 jours a fourni un petit culot qui a été analysé sur gels de SDS et qui a également été analysé par immuno-transfert. Une bande à un PM de 55 kd a été reconnue avec des anticorps spécifiques contre p17, p24 et p55. On n'a pu détecter que de très faibles quantités de produit dégradé à un PM 49-56. Sur gels teintés de Coomassie, on a pu voir une bande à 55 kd qui était pure à 20-80 %. Cette bande correspondait à celle observée sur l'"immunoblot" et était reconnue par des anticorps anti polypeptides p17, p24 et p55.

Dans l'expérimentation d'un troisième essai, la centrifugation (1ml) des milieux conditionnés de 48 heures, 3 jours et 5 jours, dans une microfuge à 12000 tpm pendant 5 à 20 minutes, a produit une bande sur gels de SDS à un PM de 55 kd qui était caractéristique pour le précurseur gag p55 de HIV comme révélé par des anticorps contre les polypeptides p17, p24 et p55 par comparaison avec la bande à 55 kd du lysat cellulaire de HIV (Molt/HTLV-III).

Dans l'expérimentation d'un quatrième essai, des milieux conditionnés de 3 jours et de 5 jours (150 ml à 1 litre, contenant 1 μg/ml d'aprotinine qui avait été ajoutée 24 heures après infection et également au moment de la récolte) ont été traités d'abord par addition de Tween 20 jusqu'à une concentration finale de 0,01 %. On y a alors ajouté une solution de polyéthylène glycol, PM 6 kd, (PEG6000) (40 % p/v dans NaCl 2M) jusqu'à concentration finale de10 % ou 5 %. Après 4 heures à 0° C, de préférence, après une nuit à 4° C, ce précipité a été centrifugé à 5000 tpm pendant 10 minutes à 4° C. Les granules de PEG ont alors été repris dans 200 μl jusqu'à 1 ml de tampon HBS (Hanks balancé en sels, Flow Laboratories, 18-102-54) contenant 0,1 % de Tween 20 et centrifugés en gradient de saccharose (20 % - 60 % dans du tampon HBS, 0,1 % de Tween 20 à 4° C contenant 10 μl d'aprotinine, Sigma Chemical Co., St. Louis, Missouri, USA) pendant environ 35minutes à 50.000 tpm dans un rotor Beckman TLA100 (Beckman Instruments, Fullerton, California, USA) à 4° C ou pendant 18 heures environ à 25000 tpm dans un rotor Beckman SW41 à 4° C. On a recueilli des frac tions de, respectivement, 0,2 à 0,5 ml de la zone d'environ 40 50 % de saccharose; ces fractions ont été congelées à -20° C et testées soit avec une prise d'antigène spécifique pour test ELISA comme de l'antisérum biotinylé -24/Ig SIDA ou antisérum SIDA/Ig de noyau de POD (HIV-1 anticore EIA, Abbott Laboratories). Un pic OD ELISA a été détecté, montrant que, sur gradients de saccharose, la protéine gag p55 migrait sous forme de particules ou de "structures agglomérées". Les fraction des pics et les fractions voisines ont été immunotransférées et révélées avec des anticorps p17, p24 ou p55. Une bande principale à PM de 55 kd dans les gels SDS-réducteurs a été détectée, correspondant à la protéine précurseur gag p55 par comparaison avec un extrait de cellules Molt.HILV-III préparées, en substance, comme décrit plus haut.

Dans l'expérimentation d'un cinquième essai, un précipité obtenu avec 5 % de PEG6000 a été préparé, en substance, comme décrit pour l'expérimentation d'un quatrième essai au départ de 150 ml d'une culture de S.f. qui avait été co-infectée avec le Baculovirus recombinant contenant le précurseur gag et avec un Baculovirus qui exprimait la protéine de manteau de HIV à un FMI de 3 à 5. Les granule de PEG6000 ont été repris dans 200 μl de tampon HBS contenant 0,1 % de Tween 20. Après centrifugation à 15000 x g pendant 1 min., le surnageant a été mélangé à 11,5 ml d'une solution de CsCl 1,5 M (0,3 volume de tampon HBS, 10mM de Tris-HCl (pH 8,0), de tétraacétate d'éthylènediamine (EDTA) 1 mM, de Tween 20 à 0,1 % et 10 μg/ml d'aprotinine. Cette suspension a été centrifugée dans un rotor Beckman 50Ti pendant environ 72 hres à 44.000 tpm à 18°. Des fractions de 300 μl chacune ont été recueillies, congelées à -20° C testées avec une prise d'antigène spécifique pour test ELISA (HIV anti core EIA). Des bandes à des densités d'environ 1,28 et 1,20 g/cm$^3$ ont été notées, la particule ressemblant au "core" ayant apparemment une densité de 1,28 g/cm$^3$.

La microscopie électronique a confirmé la présence de particules ressemblant au "pré-core" (et core) dans le milieu conditionné. La microscopie électronique à balayage a révélé des particules qui, apparemment, bourgeonnaient à la surface des cellules. La microscopie électronique par transmission immuno-or a révélé des particules qui étaient reconnues par les anticorps p24 et p55. De même, les épitopes p17, p24 et p55 étaient reconnues par marquage "immuno-or" après un court traitement de particules purifiées avec du Triton X100, dans des préparations pour microscopie électronique. Les particules étaient approximativement sphériques avec un diamètre d'environ 100 - 150 nm. Les particules manifestent des centres transparents aux électrons entourés d'un anneau opaque et une membrane extérieure et paraissent avoir la majorité des épitopes p17, p24 et p55 à la surface interne des particules.

C'est pourquoi cet exemple démontre l'expression de p55 et la formation par bourgeonnement de particules ressemblant au "pré-core" (et ressemblant au "core") de HIV comprenant la protéine précurseur gag du virus de l'immunité déficitaire. Les particules comprennent de manière prépondérante (plus de 90% du contenu protéinique total) l'entièreté de la protéine précurseur gag et sont formées en absence de séquences d'ADN d'origine virale autres que la séquence du précurseur gag et, dès lors, en absence d'autres fonctions virales comme la protéase et la transcriptase inverse du rétrovirus.

Pour démontrer que la protéine précurseur gag de HIV produite dans des cellules de S.f. est efficacement myristylée, 3 x 10⁶ cellules dans un flacon F de 25 cm² ont été marquées à 48 heures post infection avec 500 µCi d'acide myristique NET-830 (Dupont, Wilmington, Delaware, USA) pendant 18 heures après avoir été infectées avec des baculovirus contenant gag p55 à un facteur de multiplicité d'infection (FMI) de 5. Subséquemment, le milieu conditionné et les cellules ont été traités séparément pour effectuer un "Western blot" et une radioautographie sur gel de SDS. Le milieu conditionné montrait une bande principale à 55 kd qui a également été reconnue comme précurseur gag dans les "western blots" comme l'ont révélé des anticorps contre p17, p24 et p55. Deux autres bandes marquées moins importantes ont été détectées à des PM de 49-46-47 kd et ont été reconnues spécifiquement par le même groupe d'anticorps (p17, p24, p55) dans le "western blot". Des lysats cellulaires préparés dans du Triton X100 à 1 % et congelés à -20° C ont montré respectivement par radioautographie du gel de Laemli à 12,5 % et "western blot" une bande à 55 kd (et une bande mineure à 58 kd qui, apparemment, correspondait à un changement du phase de traduction, comme décrit pour le génome gag du virus rétroviral HIV-1 et des bandes plus marqués à des PM 49-47-46 et des produits de dégradation à des PM de 30-27 kd et ces dernières bandes n'étaient pas radioactives (ne contenant pas d'acide myristique).

## Exemple 3. Expression dans les cellules de mammifères

Le plasmide pRIT13002 a été introduit, pour la technique de co-précipitation au phosphate de calcium (Wigler et al., Cell 16:777 (1979), dans des cellules Cosl et CV1. A 48 hres et 110 hres après infection, les cellules et le milieu de culture ont été testés en utilisant un ELISA spécifique pour l'expression de l'antigène gag. Des extraits cellulaires (10⁶ cellules) ont été ajustés à une concentration de 1 % en Tri ton X100 ou 0,5 % DOC-NP40. L'antigène p55 a été détecté par ELISA, impliquant des anticorps polyclonaux et monoclonaux contre p17, p24 ou p55 ou en utilisant le test RIA de Dupont (NEK-040) impliquant une compétition avec le peptide p24 purifié. Les niveaux d'expression obtenus étaient entre 4 et 10 ng/ml comme le montre le test RIA de Dupont de p24.

## Exemple 4. Expression dans des cellules de levure

Le plasmide pRIT12985 a été introduit dans la souche 02276b (ura3⁻ durOʰ roc1⁻) de S. cerevisiae. Les antigènes p55 ont été détectés dans des extraits de levure (cellules en phase log-moyenne, brisées en utilisant des billes de verre ou de la zymolyase. Le précurseur gagp55 a été détecté en utilisant les tests ELISA impliquant des anticorps polyclonaux et monoclonaux contre le peptide p24 ou en utilisant le test radioimmun (RIA) de Dupont impliquant une compétition avec le peptide p24 purifié.

La protéine p55 synthétisée dans S. cerevisiae a été observée dans des "Western blots" en utilisant des anticorps monoclonaux spécifiques pour p17 ou p24 et elle avait un poids moléculaire similaire à celui de l'antigène p55 obtenu de cellules infectées par HIV.

Lorsque des extraits cellulaires ont été obtenus en absence de détergents, une fraction importante de l'antigène était retenu dans le culot après centrifugation. Cette fraction d'antigène a été largement récupérée en utilisant du Triton X100. L'utilisation de détergents pendant ou après extraction augmentait l'antigénicité comme l'ont montré les RIA. Par marquage avec de l'acide myristique tritié, le précurseur gag produit dans la levure s'est révélé myristylé et, apparemment, il était associé avec la membrane plasmatique de la cellule, comme le montre l'observation de coupes de levures au le microscope électronique (immuno-or).

Les Exemples ci-dessus démontrent l'expression de la protéine précurseur gag dans une culture de cellules eucaryotes et la formation de particules ressemblant au "pré-core" du virus de l'immunité déficitaire à la surface des cellules de Lépidoptères en utilisant un système d'expression Baculovirus. La protéine et/ou les particules ainsi préparées et purifiées et mises en formulation de vaccin pour administration parentérale à des humains en danger d'exposition à HIV, en vue de protéger les vaccinés de l'apparition de symptômes de maladie associée à une infection par HIV. Chaque dose de vaccin comprend une quantité de protéine ou de particules qui est sans danger, càd qui ne cause pas d'effets secondaires significatifs mais qui est efficace pour l'induction d'une réponse immunitaire. Par exemple, chaque dose comprend de 1 à 1000 µg, de préférence 10 à 500 µg, de protéine précurseur gag ou de particules dans un support pharmaceutiquement acceptable, p.ex. une solution aqueuse tamponnée à un pH d'environ 5 à 9, de préférence de pH 6 à 8. Le vaccin peut également comprendre un adjuvant, p.ex. de l'hydroxyde d'aluminium, un muramyl dipeptide ou une saponine comme le Quil A. Parmi les tampons utiles, il y a les tampons dérivés de cations sodium ou ammonium et les anions acétate, citrate, phosphate ou glutamate. Pour ajuster l'isotonicité ou pour stabiliser la formulation, on peut utiliser d'autres supports ou diluants pharmaceutiquement acceptables, p.ex. du chlorure de sodium, du glucose, du mannitol, de l'albumine ou du polyéthylène glycol. Le vaccin peut être lyophilisé pour la commodité du stockage et de la manipulation. Un tel vaccin est reconstitué avant administration. Alternativement, la protéine gag ou la particule peut être formulée en liposomes ou en ISCOMS par techniques connues. Une dose de vaccin exemplative comprend 100 µg de particules gag adsorbées sur hydroxyde d'aluminium dans de l'eau tamponnée à pH 7 avec de l'acétate de sodium.

Dans une forme alternative de l'invention, la protéine ou particule gag est mélangée à un ou plusieurs autres antigènes par co-expression dans la même culture cellulaire ou par co-formulation. De tels autres antigènes peuvent être d'autres antigènes de HIV, p.ex. des antigènes dérivés de la protéine d'enveloppe, gp160 ou gp120 ou peuvent être des antigènes dérivés d'un ou plusieurs autres organismes, cellules ou virus pathogènes, comme l'antigène de surface de l'hépatite B, pour conférer une protection contre le virus de l'hépatite B ou des antigènes dérivés de la glycoprotéine du virus de l'Herpes pour conférer une protection contre le virus de l'Herpes.

De préférence, le vaccin est administré par voie parentérale, p.ex. intramusculaire (im) ou souscutanée (sc) bien que d'autres voies d'administration puissent être utiles pour éliciter une réponse protective. Le vaccin est administré en dose unique ou doses multiples, p.ex. 2 à 4. L'immunoprotection peut être constatée en testant les taux d'anticorps sériques anti-gag. Ensuite, les vaccinés peuvent être vaccinés suivant la nécessité, p.ex. annuellement.

Comme réactif de diagnostic, la protéine ou les particules gag peuvent être utilisées dans tout test de diagnostic standard, comme un ELISA ou RIA, pour détecter la présence d'anticorps anti-HIV dans des échantillons cliniques. Un tel diagnostic peut être utilisé conjointement avec d'autres antigènes de HIV pour contrôler la progression de la maladie. L'utilisation de la protéine ou particule gag comme agent de diagnostic impliquera généralement la mise en contact d'un échantillon de sérum humain ou d'un animal ou d'un autre fluide du corps avec la protéine ou la particule, de préférence liée ou autrement fixée ou englobée et ensuite le test de fixation d'anticorps anti-gag du sérum ou autre échantillon contre la protéine ou les particules gag. Un tel test peut être réalisé par techniques standard, y compris en quantifiant la fixation d'anticorps anti-gag marqués subséquemment ajoutés.

La description et les exemples ci-dessus décrivent complètement l'invention et ses formes préférées de réalisation. L'invention n'est cependant pas limitée aux formes de réalisation spécifiquement décrites ici mais, plutôt, comme toutes ses améliorations, variations et modifications qui tombent dans la portée des revendications qui suivent.

Exemple 5. Construction et Expression d'un Gène Pr55 gag muté

Afin d'examiner le rôle potentiel de la N-myristylation dans l'assemblage et la formation de particules GAG extracellulaires, on a construit un mutant présentant une délétion du codon glycine (position 2). Dans ce but, on a substitué un linker oligonucléotidique syn3 synthétique au fragment BamHI-ClaI dans pRIT12982 (voir Exemple 1). Syn3 encode les acides aminés N-terminaux d'origine de la protéine GAG sauf en ce qui concerne le second codon glycine qui est supprimé. Cette cassette d'expression du mutant Pr55$^{gag}$ a été sous-clonée au site BamHI du vecteur d'expression baculo pAcYMI et on a obtenu des plaques recombinantes qui ont été sélectionnées essentiellement comme décrit dans l'Exemple 1. Le virus recombinant AcGag 31-18 qui contient la mutation (délétion du codon glycine) du gène gag a été utilisé pour infecter des cellules S.f. La protéine précurseur GAG a été synthétisée de manière efficace (test ELISA). Un marquage métabolique avec de l'acide $^3$H-myristique, essentiellement comme décrit dans l'Exemple 2 n'a révélé aucune incorporation, ce qui confirme que la suppression de la glycine N-terminale est suffisante pour abroger la myristylation de la protéine précurseur GAG. L'analyse des extraits cellulaires par Western blot (voir Exemple 1) a montré une bande prédominante de 55 kd et des produits de dégradation de poids moléculaires inférieurs. Les espèces protéiques révélées par Western blot sont les mêmes que dans le cas de la protéine Pr55$^{gag}$ de type sauvage exprimée dans les cellules S.f. Cependant, jusqu'au jour 2 post-infection, la protéine mutée (délétion de la glycine) n'a pu être détectée dans le milieu de culture, par précipitation au PEG ou par ultracentrifugation, contrairement à ce qui avait été observé avec Pr55$^{gag}$ myristylée. La protéine Pr55$^{gag}$ mutée a donc été détectée seulement dans les cellules infectées. Le processus de myristylation semble donc requis pour la libération extracellulaire du produit Pr55$^{gag}$. La microscopie électronique par balayage a révélé que la surface de la cellule était assez lisse et ne montrait pas de particules. La microscopie électronique par transmission, sur coupes minces immunodécorées des cellules infectées avec du virus recombinant Ac gag 31-18 (Myr$^-$) (24, 48 et 66 heures p.i.) a révélé que la protéine GAG nonmyristylée était exprimée de manière efficace, qu'elle se dispersait dans le cytoplasme et dans le noyau. Des particules intracellulaires ou structures particulaires morphologiquement différentes des particules extracellulaires obtenues avec le recombinant gag myristylé (AcGag7) ont été observées. Elles montrent une double structure annulaire opaque aux électrons et ne contiennent pas de double couche lipidique dérivée de la membrane cellulaire. A la membrane cellulaire on n'a pas observé de protéine GAG et on n'a observé aucune structure bourgeonnante.

Ces résultats démontrent que la myristylation des précurseurs GAG semble requise pour leur localisation à la membrane plasmatique, pour le bourgeonnement et pour la libération de particules extracellulaires. La myristylation ne paraît cependant pas requise pour l'assemblage multimérique en particules Pr55$^{gag}$. L'accumulation de Pr55$^{gag}$ non-myristylé dans le noyau (et les nucléoles) est un phénomène assez surprenant. Dans plusieurs protéines on a identifié des séquences signal spécifiques (signaux karyophiliques). Dans la séquence des acides aminés GAG on a trouvé deux séquences putatives de ce genre. Une séquence karyophilique (celle qui est la plus N-terminale) est fortement conservée entre HIV-1, HIV-2 et SIV. Ces séquences pourraient être des signaux karyophiliques fonctionnels ou accessibles seulement en absence d'acide myristique.

La migration nucléaire des produits GAG non-myristylés pourrait jouer un rôle dans la morphogénèse, p.ex. pour l'encapsidation de l'ARN génomique. Une fonction dans l'infection naturelle par HIV est également envisageable. Il faudrait pour cela que les virions infectieux contiennent au moins une molécule GAG non myristilée. Il se pourrait également que lorsque le virion infecte une cellule, et qu'il ait été débarrassé de son manteau, il y ait démyristylation et transport vers le noyau de complexes GAG-RNA/DNA (pour formation du provirus intégré dans les chromosomes).

Exemple 6. Construction et expression d'une protéine précurseur Pr55<sup>gag</sup> tronquée

Pour examiner le rôle de p16 (C-terminus) de la protéine précurseur GAG et HIV on construit un gène tronqué qui encode seulement la partie précurseur p17-p24 de GAG.

Le fragment BamHI-CfrI de GAG de pRIT13003 a été purifié et lié à la séquence oligonucléotidique synthétique

```
5' GGC CAT AAG GCA AGA GTT TTA GTT AGT TAG 3'

     3'  TA TTC CGT TCT CAA AAT CAA TCA ATC CTA G 5'
```

Le fragment résultant a été purifié sur gel et cloné au site BamHI de pAcYMI. Cette séquence linker contient l'acide aminé C-terminal de la p24 (BH10; aa 363 Ratner et al.) et deux acides aminés supplémentaires (valine et sérine). Le plasmide recombinant pAc Cfr I (p17-p24) a été utilisé pour co-transfecter des cellules S.f. avec de l'ADN wt de AcMNPV, essentiellement comme décrit plus haut (voir Exemple 1). Les plaques recombinantes ont été criblées comme décrit dans l'Exemple 1.

Un virus Ac CfrI recombinant a été utilisé pour infecter des cellules S.f. Un polypeptide GAG tronqué a été détecté au poids moléculaire attendu de 41 Kd : il réagissait (Western blot) avec les monoclonaux p17 et 24. Le produit p17-24 était, de manière prédominante, exprimé à l'intérieur des cellules mais on a pu détecter une petite quantité de produit p17-24 extracellulaire lors de l'analyse du milieu conditionné. Une précipitation au PEG et une ultracentrifugation du milieu de culture des cellules infectées par AcCfrI n'a pas permis de détecter de protéine p17-24. L'analyse au microscope électronique n'a montré aucune évidence de bourgeonnement ou de particules GAG extracellulaires. De grandes protubérances de 1-4 μm de longueur en forme de structures tubulaires connectées longitudinalement à la surface de la membrane cellulaire ont pu être détectées au début de l'infection. Un marquage immuno-or a montré que la protéine GAG (p17-24) tronquée était localisée à la membrane cellulaire et à la périphérie de ces extensions tubulaires mais on n'a pas pu détecter de structures "annulaires" opaques aux électrons, typiques de la particule Pr55<sup>gag</sup>. Ces résultats suggèrent qu'au moins une partie du polypeptide p16 est nécessaire pour l'assemblage correct de la particule.

Le motif "Zn finger(?)" est peut-être essentiel pour l'assemblage correct de molécules Pr55<sup>gag</sup> en particules bourgeonnantes. Un mutant de la cassette CfrI (p17-p24 non myrystylé) comportant une délétion du codon glycine (position 2) a été construit en échangeant le fragment EcoRV-PstI de 669 pb de la cassette codant pour Pr55<sup>gag</sup> non-myristylé (de pAcGag 31-38) avec le long fragment EcoRV-PstI de ± 9400 pb de pAc CfrI.

La protéine p17p24 non myristylée est efficacement produite mais ne donne pas lieu à la formation de protubérances membranaires. L'immunodécoration des coupes de cellules montre qu'elle est disséminée à l'intérieur de celles-ci.

Exemple 7. Construction et expression d'un gène gagpol et maturation des polypeptides produits

Pour exprimer les produits GAG-POL, on a introduit la plus grande partie (± 80 %) du gène pol dans le vecteur de transfert de baculovirus portant la cassette d'expression de Pr55<sup>gag</sup> (pRIT13003). Le fragment d'ADN portant le gène pol est un fragment de restriction (clone BH10) de BglII (2093)-EcoRI (4681). Un fragment d'ADN synthétique (poly-stop)

```
5' AAT TCC TAA CTA ACT AAG 3'

     3' GGA TTG AT   TGA TTC CTA G     5'
```

a été ajouté au site EcoRI.

Le plasmide de transfert de baculovirus résultant, LE-8-4, a été employé dans une expérience de cotransfection pour générer des plaques recombinantes, essentiellement comme décrit dans l'Exemple 1.

Avec cette construction recombinante, on s'attend à la production de pr55<sup>gag</sup> aussi bien que du produit GAG-POL résultant du changement de phase de lecture HIV spécifique, lesquels seront ensuite maturés par la protéase (voir Kramer et al., 1985).

Dans le milieu de culture des cellules S.f. infectées par un tel virus recombinant vAcGag8-5 on n'a détecté aucun produit gag ou gag-pol lorsque le milieu conditionné a été analysé par Western blot ou après précipitation au PEG.

Cependant, les extraits cellulaires montraient une forte bande double à 24 Kd et une bande à 17 Kd qui réagissait avec des anticorps monoclonaux de p24 et p17 (Western blot). De très petites quantités de la bande de précurseur Pr55<sup>gag</sup> et des bandes intermédiaires de 41 kd (46 kd) ont également pu être détectées (Western blot). Ceci indique que la protéase portée par la protéine de fusion GAG-POL est active et que le changement de phase de traduction HIV-spécifique est fonctionnel dans les cellules S.f. Les polypeptides p17 et 24 et des intermédiaires (41 kd, 46-49 kd, 55 kd) ont été détectés. Le grand précurseur GAG-POL n'a pas été détecté avec nos anticorps p55 ou p17, p24 (il est probablement très peu abondant).

La microscopie électronique a montré, à de rares occasions, quelques particules bourgeonnant au niveau de la membrane cellulaire. Ces particules paraissaient être morphologiquement similaires aux particules Pr55$^{gag}$ décrites plus haut. Des expériences de co-infection avec des virus recombinants portant respectivement les gènes gag et gag-pol n'ont pas amené la détection de particules à structure de noyau (p24) plus condensée, typique de la particule rétrovirale (de HIV) mature.

Exemple 8. Construction et expression du gène de Pr57$^{57}$gag de SIV dans les cellules S.f.

Le gène gag du virus de l'immunité déficitaire de singe (SIV) a été sous-cloné de SIV$_{mac}$-BK28 (reçu de J. Mullins). Un fragment KpnI de 3504 pb du génome de pBK28 (nucléotides 1212 à 4716) a été sous-cloné dans pUC8. Deux fragments internes du gène gag, le fragment 5′ FnuDII (1201) - Pst (1959) et le fragment 3′ PstI (1959) - HphI (2803) ont été purifiés et les linkers oligonucléotidiques linker 1

```
GAT CC ACC ATG GGC              et linker 3
    G TGG TAC CCG
```

TGCTGCACCTCAATTCTCTCTTTGGAGGAGACCAGTAGAGATCTGGTAC

AACGACGTGGAGTTAAGAGAGAAACCTCCTCTGGTCATCTCTAGAC

ont été liés à des fragments gag adéquats pour reconstituer l'entièreté du gène précurseur gag. Dans une expérimentation séparée, un linker

```
2 GATCC ACC ATG GCC
      G TGG TAC CGG
```

a été utilisé pour introduire une mutation dans le second codon GGC (Gly) → GCC (Ala). Les différentes constructions ont été clonées dans des vecteurs et vérifiées par séquençage. Le fragment 5′ terminal (BamHI-PstI) et le fragment 3′ (PstI-BglII) ont été isolés et clonés dans le vecteur de transfert de baculovirus PACYMI digéré avec BamHI et traité à la phosphatase alcaline. Le plasmide pAC gag Myr$^+$ contient le gène de gag SIV natif et le pAC gag Myr$^-$ contient le gène muté (Gly → Ala). Des cellules S.f; ont été transfectées avec un mélange d'ADN viral de AC MNPV (1 μg) et des plasmides de transfert recombinants respectifs (50 μg), essentiellement comme décrit dans l'Exemple 1. Les plaques recombinantes ont été criblées et sélectionnées comme décrit dans l'Exemple 1.

Le polypeptide précurseur gag Pr57$^{gag}$ de SIV a été synthétisé avec succès dans des cellules d'insecte infectées (observé par Western blot) en utilisant de l'antisérum de lapin contre SIV (virus SIV-BK28 purifié avec un gradient de métrizamide) ou un monoclonal dirigé contre l'extrémité COOH du polypeptide p24 de HIV, lequel s'avère également reconnaître la protéine de core de SIV).

Dans une seconde expérience, il a été démontré que la protéine Pr 55$^{gag}$ de SIV produite dans des cellules S.f. était efficacement myristylée, par analyse sur SDS-PAGE et par radioautographie. Par contre, dans le cas de la protéine mutée (glycine → alanine), aucune myristylation n'a pu être détectée.

Comme dans le cas de la protéine Pr55$^{gag}$ de HIV, on a également observé la formation et la libération de particules dans le milieu conditionné lors de l'analyse des cultures infectées [par ultracentrifugation, gradient de saccharose et microscopie (TEM et SEM)]. On a observé des particules gag Pr57$^{gag}$ de SIV similaires à celles obtenues par l'expression de Pr55$^{gag}$ de HIV dans des cellules S.f. Les particules gag extracellulaire forment des structures qui croissent au niveau de la membrane cellulaire, qui s'assemblent en bourgeonnements typiques, qui ressemblent fortement aux particules bourgeonnantes du virus non mature. Les particules Pr57$^{gag}$ de SIV comme les particules Pr55$^{gag}$ de HIV avaient environ 100-120 nm de diamètre et montraient un centre transparent aux électrons entouré d'un anneau foncé et épais et une double couche lipidique extérieure. Des expérimentations réalisées avec le mutant (Gly → Ala) non myristylé de SIV ont confirmé les observations faites avec le mutant Pr55$^{gag}$ non myristylé de HIV, à savoir que la N-myristylation est essentielle pour le bourgeonnement et la formation de particules extracellulaires. La différence entre la protéine précurseur GAG de HIV et de SIV est que cette dernière forme également des particules intracellulaires et une structure particulière lors de l'expression de la protéine GAG myristylée. Ceci peut être expliqué comme suit : le taux d'expression est environ 3 fois plus élevé que le taux d'expression de gag de HIV et il se peut que toutes les molécules Pr57$^{gag}$ de SIV ne soient pas myristylées. On a pu déceler également plus de produits de dégradation, spécialement une bande protéinique p27 myristylée.

Il est possible que les structures cellulaires d'environ 40 nm de diamètre et parfois jusqu'à 1 μm de long - qui sont observées à un stade avancé ces infections - soient composées au moins en partie de ces produits de dégradation de gag. Ceci pourrait ressembler à l'assemblage du noyau (?) de p24 en structures tubulaires observées dans de rares cas de maturation de noyau de rétrovirus. On a également observé (C. Debouck, comm. personn.) des structures tubulaires contenant la protéine p24 lorsque la protéine de noyau p24 de HIV-1 est exprimée dans E. coli. Une partie des particules intracellulaires observées avec le recombinant du précurseur de gag de SIV natif prend forme près de la membrane cellulaire où elles paraissent se différencier en particules ressemblant au virus et bourgeonnant comme décrit plus haut. Ce processus rappelle la maturation virale de rétrovirus du type D qui implique des particules intracellulaires intermédiaires de type A.

**Revendications**

1. Molécule d'ADN recombinant comprenant une séquence d'ADN qui code pour l'entièreté de la protéine précurseur gag du virus de l'immunité déficitaire et qui est dépourvue des séquences flanquantes se présentant naturellement en 5′ et en 3′, liée de manière opératoire à un élément de régulation qui fonctionne dans des cellules eucaryotes.

2. Molécule d'ADN recombinant de la revendication 1 dans laquelle l'élément de régulation est un élément qui fonctionne dans les cellules de levure, d'insecte ou de mammifère et la protéine précurseur gag est la protéine précurseur gag de HIV.

3. Molécule d'ADN recombinant de la revendication 2 dans laquelle l'élément de régulation est un élément qui fonctionne dans des cellules de Lépidoptères.

4. Molécule d'ADN recombinant de la revendication 3 dans laquelle l'élément de régulation comprend le promoteur du gène de polyhédrine.

5. Baculovirus recombinant comprenant la molécule d'ADN recombinant de la revendication 2, 3 ou 4.

6. Cellule d'insecte infectée par le Baculovirus recombinant de la revendication 5.

7. Cellule d'insecte de la revendication 6 qui est une cellule de Lépidoptère.

8. Cellule d'insecte de la revendication 6 qui est une cellule de Spodoptera frugiperda.

9. Molécule d'ADN recombinant de la revendication 2 dans laquelle l'élément de régulation est un élément qui fonctionne dans des cellules de Drosophile.

10. Cellule de Drosophile transformée avec la molécule d'ADN recombinant de la revendication 9.

11. Molécule d'ADN recombinant de la revendication 2 dans laquelle la région de régulation est une région qui fonctionne dans des cellules de mammifère.

12. Virus de la vaccine recombinant comprend la molécule d'ADN recombinant de la revendication 11.

13. Cellule de mammifère comprenant la molécule d'ADN recombinant de la revendication 11.

14. Cellule de mammifère infectée avec le virus recombinant de la vaccine de la revendication 12.

15. Cellule de mammifère de la revendication 13 qui est choisie dans le groupe comprenant les cellules CHO, les cellules COS-7, les cellules NIH-3T3, les cellules CV1, les cellules de myélome de la souris et du rat, les cellules HAK, les cellules Vero, les cellules Hela, les cellules WI38, les cellules PRC-5 et les cellules de hématosarcome du poulet.

16. Molécule d'ADN recombinant de la revendication 2 dans laquelle la région de régulation est une région qui fonctionne dans une levure.

17. Molécule d'ADN recombinant de la revendication 16 dans laquelle l'élément de régulation comprend le promoteur CUP1, TDH3, PGK, ADH, PH05 ou ARG3.

18. Cellule de levure recombinante comprenant la molécule d'ADN recombinant de la revendication 16.

19. Cellule de S. cerevisiae comprenant la molécule d'ADN recombinant de la revendication 17.

20. Molécule d'ADN recombinant pour l'expression dans des cellules de Lépidoptères et la formation d'une particule qui est immunologiquement similaire aux particules gag authentiques immatures du virus de l'immunité déficitaire, laquelle molécule comprend une séquence d'ADN qui code pour l'entièreté ou une portion de la protéine précurseur gag du virus de l'immunité déficitaire ou pour une protéine hybride ayant l'entièreté ou une portion d'une protéine précurseur gag de l'immunité déficitaire, liée de manière opératoire à un élément de régulation qui fonctionne dans des cellules de Lépidoptères.

21. Molécules d'ADN recombinant de la revendication 20, pour l'expression et la formation d'une particule constituée de manière prépondérante de protéines précurseur gag de HIV, laquelle protéine code pour l'entièreté de la protéine précurseur gag de HIV et dépourvue d'autres fonctions de HIV.

22. Molécule d'ADN recombinant comprenant une séquence codant pour une protéine précurseur gag du virus de l'immunité déficitaire liée de manière opératoire à une région de régulation qui fonctionne dans les cellules de Lépidoptères.

23. Molécule d'ADN recombinant de la revendication 22 dans laquelle la séquence qui code pour l'entièreté de la protéine précurseur gag de HIV est dépourvue d'autres fonctions de HIV.

24. Molécules d'ADN recombinant de la revendication 20, 21, 22 ou 23 dans laquelle l'élément de régulation comprend le promoteur du gène de la polyhédrine.

25. Baculovirus recombinant comprenant la molécule d'ADN recombinant de la revendication 20 ou 22.

26. Baculovirus recombinant comprenant la molécule d'ADN recombinant de la revendicaiton 24.

27. Cellule de Lépidoptère infectée avec le Baculovirus recombinant de la revendication 25.

28. Cellule de Spodoptera frugiperda infectée par le Baculovirus recombinant de la revendication 26.

29. Protéine précurseur gag produite par la culture de cellules de la revendication 6.

30. Protéine précurseur gag produite par la culture de cellules de la revendication 13 ou 18.

31. Protéine précurseur gag produite par la culture de cellules de la revendication 27.

32. Particule formée de protéines précurseur gag isolées d'un milieu conditionné au départ d'une culture de cellules de la revendication 6.

33. Particule formée de protéine précurseur gag produite par la culture de cellules de la revendication 27.

34. Particule immunogène formée de protéines précurseur gag produite par des cellules d'eucaryotes recombinantes, laquelle particule est immunologiquement similaire aux particules gag du virus de l'immunité déficitaire authentique.

35. Particule immunogène de la revendication 34 qui comprend de manière prédominante les protéines précurseur gag de HIV qui sont reconnuee par des anticorps anti-p16, anti-p24 et anti-p17 et qui est dépourvue des fonctions virales requises pour la maturation et la réplication du virus.

36. Vaccin comprenant la protéine précurseur gag produite par des cellules d'encaryote recombinantes.

37. Vaccin comprenant des particules formées de protéines précurseur gag produites par des cellules d'eucaryote recombinantes.

38. Méthode pour recueillir des données utiles dans le diagnostic de l'exposition d'un animal à un virus de l'immunité déficitaire acquise qui comprend la mise en contact d'un échantillon de sérum ou d'un autre fluide corporel de l'animal avec une protéine précurseur gag de la revendication 29, 30, 31 ou 32.

39. Méthode pour recueillir des données utiles dans le diagnostic de l'exposition d'un animal à un virus de l'immunité déficitaire acquise qui comprend la mise en contact d'un échantillon de sérum ou d'autre fluide corporel de l'animal avec la particule immunogène de la revendication 34 ou 35.

40. Protéine précurseur gag suivant l'une quelconque des revendications 29, 30, 31 et 32 pour une utilisation comme agent vaccinal.

41. protéine précurseur gag suivant l'une quelconque des revendications 29, 30, 31 et 32 pour une utilisation comme agent vaccinal pour conférer à des humains une protection contre une infection par HIV.

42. Protéine précurseur gag suivant l'une quelconque des revendications 29, 30, 31 et 32 pour une utilisation dans la fabrication d'un vaccin pour conférer à des humains une protection contre une infection par HIV.

43. Particule immunogène de la revendication 34 ou 35 pour une utilisation comme agent vaccinal.

44. Particule immunogène de la revendication 34 ou 35 pour une utilisation comme agent vaccinal pour conférer à des humains une protection contre une infection par HIV.

45. Particule immunogène de la revendication 34 ou 35 pour une utilisation dans la fabrication d'un vaccin pour conférer à des humains une protection contre une infection par HIV.

## Revendications pour les Etats contractants suivants: ES, GR

1. Molécule d'ADN recombinant comprenant une séquence d'ADN qui code pour l'entièreté de la protéine précurseur gag du virus de l'immunité déficitaire et qui est dépourvue des séquences flanquantes se présentant naturellement en 5' et en 3', liée de manière opératoire à un élément de régulation qui fonctionne dans des cellules eucaryotes.

2. Molécules d'ADN recombinant de la revendication 1 dans laquelle l'élément de régulation est un élément qui fonctionne dans les cellules de levure, d'insecte ou de mammifère et la protéine précurseur gag est la protéine précurseur gag de HIV.

3. Molécule d'ADN recombinant de la revendication 2 dans laquelle l'élément de régulation est un élément qui fonctionne dans des cellules de Lépidoptères.

4. Molécule d'ADN recombinant de la revendication 3 dans laquelle l'élément de régulation comprend le promoteur du gène de polyhédrine.

5. Baculovirus recombinant comprenant la molécule d'ADN recombinant de la revendication 2, 3 ou 4.

6. Cellule d'insecte infectée par le Baculovirus recombinant de la revendication 5.

7. Cellule d'insecte de la revendication 6 qui est une cellule de Lépidoptère.

8. Cellule d'insecte de la revendication 6 qui est une cellule de Spodoptera frugiperda.

9. Molécule d'ADN recombinant de la revendication 2 dans laquelle l'élément de régulation est un élément qui fonctionne dans des cellules de Drosophile.

10. Cellule de Drosophile transformée avec la molécule d'ADN recombinant de la revendication 9.

11. Molécule d'ADN recombinant de la revendication 2 dans laquelle la région de régulation est une région qui fonctionne dans des cellules de mammifère.

12. Virus de la vaccine recombinant comprenant la molécule d'ADN recombinant de la revendication 11.

13. Cellule de mammifère comprenant la molécule d'ADN recombinant de la revendication 11.

14. Cellule de mammifère infectée avec le virus recombinant de la vaccine de la revendication 12.

15. Cellule de mammifère de la revendication 13 qui est choisie dans le groupe comprenant les cellules CHO, les cellules COS-7, les cellules NIH-3T3, les cellules CV1, les cellules de myélome de la souris et du rat, les cellules HAK, les cellules Vero, les cellules Hela, les cellules WI38, les cellules PRC-5 et les cellules de hématosarcome du poulet.

16. Molécule d'ADN recombinant de la revendication 2 dans laquelle la région de régulation est une région qui fonctionne dans une levure.

17. Molécule d'ADN recombinant de la revendication 16 dans laquelle l'élément de régulation comprend le promoteur CUP1, TDH3, PGK, ADH, PH05 ou ARG3.

18. Cellule de levure recombinante comprenant la molécule d'ADN recombinant de la revendication 16.

19. Cellule de S. cerevisiae comprenant la molécule d'ADN recombinant de la revendication 17.

20. Molécule d'ADN recombinant pour l'expression dans des cellules de Lépidoptères et la formation d'une particule qui est immunologiquement similaire aux particules gag authentiques immatures du virus de l'immunité déficitaire, laquelle molécule comprend une séquence d'ADN qui code pour l'entièreté ou une portion de la protéine précurseur gag du virus de l'immunité déficitaire ou pour une protéine hybride ayant l'entièreté ou une portion d'une protéine précurseur gag de l'immunité déficitaire, liée de manière opératoire à un élément de régulation qui fonctionne dans des cellules de Lépidoptères.

21. Molécule d'ADN recombinant de la revendication 20, pour l'expression et la formation d'une particule constituée de manière prépondérante de protéines précurseur gag de HIV, laquelle protéine code pour l'entièreté de la protéine précurseur gag de HIV et dépourvue d'autres fonctions de HIV.

22. Molécule d'ADN recombinant comprenant une séquence codant pour une protéine précurseur gag du virus de l'immunité déficitaire liée de manière opératoire à une région de régulation qui fonctionne dans les cellules de Lépidoptères.

23. Molécule d'ADN recombinant de la revendication 22 dans laquelle la séquence qui code pour l'entièreté de la protéine précurseur gag de HIV est dépourvue d'autres fonctions de HIV.

24. Molécule d'ADN recombinant de la revendication 20, 21, 22 ou 23 dans laquelle l'élément de régulation comprend le promoteur du gène de la polyhédrine.

25. Baculovirus recombinant comprenant la molécule d'ADN recombinant de la revendication 20 ou 22.

26. Baculovirus recombinant comprenant la molécule d'ADN recombinant de la revendication 24.

27. Cellule de Lépidoptère infectée avec le Baculovirus recombinant de la revendication 25.

28. Cellule de Spodoptera frugiperda infectée par le Baculovirus recombinant de la revendication 26.

29. Méthode pour préparer un vaccin pour protéger un humain contre une maladie causée par une infection par HIV qui comprend la culture de cellules eucaryotes recombinantes qui ont été transformées avec une molécule d'ADN recombinant comprenant une séquence d'ADN qui code pour l'entièreté de la protéine précurseur gag de HIV et qui est dépourvue des séquences flanquantes 5′ et 3′ qui se présentent naturellement, liées de manière opératoire à un élément de régulation qui fonctionne dans les cellules eucaryotes; l'isolement des particules de protéine précurseur gag ainsi produites et l'association des particules de protéine précurseur gag isolées à un support pharmaceutiquement acceptable.

30. Protéine précurseur gag produite par la culture de cellules de l'une quelconque des revendications 6, 13 ou 18 pour une utilisation comme agent vaccinal.

31. Protéine précurseur gag produite par la culture de cellules de la revendication 6, 13 ou 18 pour une utilisation comme agent vaccinal pour conférer à des humains une protection contre une infection par HIV,

32. Protéine précurseur gag produite par la culture de cellules de la revendication 6, 13 ou 18, pour une utilisation dans la fabrication d'un vaccin pour conférer à des humains une protection contre une infection par HIV.

33. Particules gag immunogène comprenant une protéine précurseur gag produite par des cellules encaryotes recombinantes, laquelle particule est immunologiquement similaire aux particules gag du virus de l'immunité déficitaire acquise authentiques pour une utilisation comme agent vaccinal.

34. Particule gag immunogène comprenant une protéine précurseur gag produite par des cellules encaryotes recombinantes, laquelle particule est immunologiquement similaire aux particules gag du virus de l'immunité déficitaire acquise authentiques pour une utilisation comme agent vaccinal pour conférer à des humains une protection contre une infection par HIV.

35. Particule gag immunogène comprenant une protéine précurseur gag produite par des cellules eucaryotes recombinantes, laquelle particule est immunologiquement similaire aux particules gag du virus de l'immunité déficitaire acquise authentiques pour une utilisation dans la fabrication d'un vaccin pour conférer à des humains une protection contre une infection par HIV.

36. Procédé pour préparer une molécule d'ADN recombinant pour exprimer une protéine gag du virus de l'immunité déficitaire acquise qui comprend la ligation d'une séquence d'ADN qui code pour l'entièreté de la protéine précurseur gag du virus de l'immunité déficitaire acquise et qui est dépourvue des séquences flanquantes en 5′ et en 3′ qui se présentent naturellement, à un élément de régulation qui fonctionne dans des cellules eucaryotes.

37. Procédé de la revendication 36 dans lequel l'élément de régulation est un élément de régulation qui fonctionne dans une levure, des cellules d'insecte ou de mammifère et la protéine précurseur gag est la protéine précurseur de HIV.

38. Procédé de la revendication 37 dans lequel l'élément de régulation est un élément de régulation qui fonctionne dans des cellules de Lépidoptères.

39. Procédé de la revendication 38 dans lequel l'élément de régulation comprend le promoteur du gène de polyhédrine.